Europäisches Patentamt

European Patent Office

Office européen des brevets

(11) Veröffentlichungsnummer : **0 064 634**
**B1**

(12) **EUROPÄISCHE PATENTSCHRIFT**

(45) Veröffentlichungstag der Patentschrift :
25.07.84

(51) Int. Cl.³ : **C 07 C 55/02**, C 07 C 51/285,
C 07 C 51/34

(21) Anmeldenummer : **82103381.8**

(22) Anmeldetag : **21.04.82**

(54) **Verfahren zur Herstellung von 3,3-Dimethylglutarsäure.**

(30) Priorität : **12.05.81 CH 3061/81**

(43) Veröffentlichungstag der Anmeldung :
**17.11.82 Patentblatt 82/46**

(45) Bekanntmachung des Hinweises auf die Patenterteilung : **25.07.84 Patentblatt 84/30**

(84) Benannte Vertragsstaaten :
**AT BE CH DE FR GB IT LI LU NL SE**

(56) Entgegenhaltungen :
**DE-B- 2 303 925**

(73) Patentinhaber : **LONZA AG**
**Gampel/Wallis (CH)**

(72) Erfinder : **Lehky, Pavel, Dr.**
**Dammweg 13**
**Naters (Kanton Wallis) (CH)**
Erfinder : **Hardt, Peter, Dr. Dipl.-Chem.**
**Bäretstrasse 8**
**Visp (Kanton Wallis) (CH)** .

(74) Vertreter : **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

## Beschreibung

Die Erfindung betrifft ein Verfahren zur Herstellung von 3,3-Dimethylglutarsäure aus Isophoron.

3,3-Dimethylglutarsäure und ihre Ester stellen Zwischenprodukte bei der Herstellung von Pestiziden (DE-OS 28 13 341) dar. Ferner sind sie als Zusatz für Schmieröl geeignet (US-PS 2 971 915). Für die Herstellung von 3,3-Dimethylglutarsäure sind aus der Literatur verschiedene Methoden bekannt. So wird nach W.T. Smith und G.L.McLeod, Org. Synthesis 31, 40 (1951) durch Oxidation von Dimedon (5,5-Dimethyl-1,3-cyclohexandion) mit Natriumhypochlorit 3,3-Dimethylglutarsäure erhalten. F.B. Thole und J.F. Thorpe, J. Chem. Soc. 99, 422 (1911) erhalten Dimethylglutarsäure aus Aceton und Cyanacetamid. W.H. Perkin jun. und J.F. Thorpe, J. Chem. Soc. 75, 48 (1899), stellen Dimethylglutarsäure aus Dimethylacrylester und Cyanessigester her.

Alle diese bekannten Verfahren haben die Nachteile, dass sie entweder von teuren Edukten ausgehen oder über mehrere Synthesestufen verlaufen oder niedere Ausbeuten bringen, viel Abfallsalz als Nebenprodukt produzieren oder stark verunreinigtes Endprodukt liefern.

Ziel der vorliegenden Erfindung ist es, 3,3-Dimethylglutarsäure, ausgehend von einem billigen Edukt, auf einfache Weise in hoher Reinheit zu produzieren, so dass keine weiteren Reinigungsverfahren nachgeschaltet werden müssen.

Erfindungsgemäss wird dies dadurch erreicht, dass man von Isophoron ausgeht, dieses in ein Ozonanlagerungsprodukt überführt, dieses in die 3,3-Dimethyl-5-oxo-hexansäure überführt und aus letzterer mit Hilfe einer Wasserstoffperoxidbehandlung die 3,3-Dimethylglutarsäure herstellt.

Die erfindungsgemässe Umsetzung kann diskontinuierlich oder kontinuierlich durchgeführt werden. Vorzugsweise wird die erste Stufe, nämlich die Ozonierung und die Hydrolyse, zur 3,3-Dimethyl-5-oxo-hexansäure kontinuierlich durchgeführt und die Oxohexansäure isoliert. In einer weiteren Stufe wird die Umsetzung mit $H_2O_2$ zum Endprodukt vorgenommen.

Die erste Stufe, also die Ozonierung und Hydrolyse, kann ohne oder in Gegenwart von artfremden Lösungsmitteln durchgeführt werden. Wird ohne artfremde Lösungsmittel gearbeitet, so kann Isophoron selbst als Lösungsmittel dienen. Werden artfremde Lösungsmittel verwendet, kommen zweckmässig Alkohole, wie Methanol, Ethanol, Isopropanol, organische Säuren, wie Essigsäure, Ameisensäure, Ester, wie Essigester, halogenierte Kohlenwasserstoffe, wie Methylenchlorid, Tetrachlorkohlenstoff, aliphatische, cycloaliphatische oder aromatische Kohlenwasserstoffe, wie Petroläther und Cyclohexan, zum Einsatz. Diese Lösungsmittel können allein oder im Gemisch eingesetzt werden, z. B. Methanol und Essigester oder Essigsäure und Essigester.

Das Verfahren der Erfindung kann aber auch in Gegenwart von Wasser durchgeführt werden. Da Isophoron nicht in Wasser löslich ist, liegt es in Wasser suspendiert vor und die Reaktion erfolgt in Suspension.

Die Ozonolyse wird vorteilhaft bei Temperaturen von − 80 bis + 40 °C durchgeführt. Aus praktischen Gründen wird vorteilhafterweise eine Temperatur von − 20 bis + 20 °C gewählt.

Die Reaktionszeit hängt wesentlich von der Leistung des Ozongenerators ab. Mittlere Reaktionszeiten liegen etwa bei wenigen Minuten bis 6 Stunden.

Die Hydrolyse des Ozonanlagerungsproduktes wird bevorzugt mit Hilfe von heissem, vorzugsweise kochendem Wasser, durchgeführt. Die dabei entstehende 3,3-Dimethyl-5-oxo-hexansäure wird vorteilhaft isoliert. Die Isolierung kann zweckmässig durch Zusatz von anorganischen Salzen, wie NaCl, KCl, Phosphate, Sulfate, erreicht werden. Vorteilhaft wird die Hydrolyse direkt in der Salzlösung durchgeführt.

Anschliessend wird die 3,3-Dimethyl-5-oxo-hexansäure in einer zweiten Stufe mit $H_2O_2$ behandelt. Vorteilhaft wird dafür eine 20 bis 70 %ige wässrige $H_2O_2$-Lösung eingesetzt und in Gegenwart einer starken Säure gearbeitet. Als starke Säuren kommen die Säuren in Frage, die im Wasser vollständig dissoziert sind. Vorteilhaft werden vor allem Schwefelsäure und Phosphorsäure verwendet.

Die Temperatur, die bei der $H_2O_2$-Behandlung angewendet wird, liegt insbesondere bei 15 bis 70 °C, vorzugsweise 30 bis 50 °C.

Für die. Durchführung der Reaktion wird in einem ersten Schritt ein Gemisch von Wasser, Wasserstoffperoxid und einer starken Säure hergestellt, indem man eine Lösung von Wasserstoffperoxid, eine Säure oder eine wässrige Lösung einer Säure und gegebenenfalls Wasser vereinigt.

Spezielle Ausführungsformen der zweiten Stufe sind wie folgt : Wasserstoffperoxid wird als wässrige Lösung eingesetzt, wobei die Konzentration in einem weiten Bereich von 20 % bis ca. 85 % variieren kann. Bevorzugt werden technisch erhältliche Konzentrationen von 30 % und 70 % $H_2O_2$ in Wasser.

Die starke Säure kann in reiner Form oder als wässrige Lösung eingesetzt werden. Im Falle, wo als starke Säure Schwefelsäure verwendet wird, wird bevorzugt technische, konzentrierte Schwefelsäure eingesetzt.

Die Mengen und die Konzentrationen von Wasserstoffperoxidlösung, starker Säure und gegebenenfalls Wasser werden so gewählt, dass auf 1 Mol 3,3-Dimethyl-5-oxohexansäure 5-10 Mol Säure, 4-10 Mol Wasserstoffperoxid und 20-40 Mol Wasser vorliegen.

Ein besonders vorteilhaftes Verhältnis ist 8 Mol Schwefelsäure, 7 Mol Wasserstoffperoxid und 33 Mol Wasser, welches für die Oxidation von 1 Mol 3,3-Dimethyl-5-oxo-hexansäure eingesetzt wird.

Nach Beendigung der Reaktion wird auf tiefere Temperaturen, vorteilhafterweise auf − 10 bis 0 °C, abgekühlt und die auskristallisierende Dimethylglutarsäure abgetrennt. Die so erhaltene 3,3-Dimethylglutarsäure ist rein und muss nicht besonders gereinigt werden.

Beispiel 1

1. Stufe

Eine Lösung von 200 g Isophoron in 1 500 g Methanol wurde während 16 Stunden oben in eine Glockenbodenkolonne eingeleitet. Von unten wurde Sauerstoff, der ca. 4 % Ozon enthält, eingeleitet. Flüssigphase und Gasphase bewegten sich somit im Gegenstrom.

Die Kolonne wurde auf der Aussenseite durch einen Kühlmantel gekühlt, wobei das Kühlmedium 0 bis 2 °C hatte. Die von der Kolonne unten ablaufende Lösung wurde kontinuierlich in eine Lösung aus 82,5 g primären Natriumphosphat und 400 g Wasser eingeleitet. Diese Wasserphase wurde auf 90 °C gehalten. Das Methanol destillierte kontinuierlich ab.

Nach beendeter Ozonolyse wurde die Phosphatlösung auf Raum-temperatur abgekühlt. Die obere Schicht bestand aus 258,5 g feuchter 3,3-Dimethyl-5-oxo-hexansäure ; dieses feuchte Produkt hatte laut Gaschromatographie einen Gehalt von 88 %, entsprechend 227,5 g 100 %igem Produkt. Dies entsprach einer Ausbeute von 99,4 %, bezogen auf eingesetztes Isophoron.

Als Verunreinigung fand man nur Wasser, das leicht entfernt werden konnte.

2. Stufe

20 g konzentrierte Schwefelsäure und 20,0 g 30 %iges Perhydrol wurden unter Kühlung vereinigt. Diese Mischung wurde auf 30 bis 35 °C abgekühlt. Hierzu tropfte man unter Rühren 4,0 g Dimethyl-oxo-hexansäure so schnell zu, dass die Temperatur bei 35 °C blieb. Nach beedeter Zugabe erhöhte man die Temperatur auf 50 °C und hielt 14 Stunden bei dieser Temperatur.

Nach beendeter Reaktion wurde auf Raumtemperatur gekühlt. Die restlichen Peroxide wurden mit Natriumsulfit zersetzt. Der pH der Lösung wurde mit Natronlauge auf 1,5 eingestellt. Hierauf extrahierte man dreimal mit je 25 ml Aether. Die Aetherphasen wurden vereinigt, getrocknet und zur Trockene eingedampft.

Man erhielt 3,97 g einer Dimethylglutarsäure mit einem Gehalt von 82,6 %. Dies entsprach einer Ausbeute von 81,8 %, bezogen auf eingesetzte Dimethyl-oxo-hexansäure.

Beispiel 2

1. Stufe

Eine Suspension von 15,8 g Isophoron in 110 ml Wasser wurde auf 0 °C abgekühlt. Dann wurde so lange ein Sauerstoffstrom, der ca. 4 % Ozon enthält, eingeleitet, bis die Suspension mit Ozon gesättigt war.

Das Ganze wurde nun auf 95 °C erwärmt und 3 Stunden lang unter Rückfluss gekocht. Die gebildete 3,3-Dimethyl-5-oxo-hexansäure wurde anschliessend durch Abdampfen des Wassers unter Vakuum isoliert.

Man erhielt 18,4 g einer leicht gelben Flüssigkeit. Dieses Produkt hatte laut Gaschromatographie einen Gehalt von 92,3 %, entsprechend 16,9 g 100 %igem Produkt. Dies entsprach einer Ausbeute von 93,8 %, bezogen auf eingesetztes Isophoron. Als Verunreinigung fand man nur Wasser, das leicht entfernt werden konnte.

2. Stufe

Wie in Beispiel 1.

Beispiel 3

1. Stufe

Eine Lösung von 16 g Isophoron in 100 g Dichlormethan wurde auf 0 °C abgekühlt. Durch diese Lösung wurde so lange ein Sauerstoffstrom, der ca. 4 % Ozon enthält, geleitet, bis die Lösung mit Ozon gesättigt war.

Dann wurde die ozonolysierte Lösung in 150 ml kochendes Wasser eingetropft, das Dichlormethan wurde abdestilliert und die wässrige Lösung noch 3 Stunden lang gekocht. Die gebildete 3,3-Dimethyl-5-oxo-hexansäure wurde durch Einengen im Vakuum isoliert.

Man erhielt 21,65 g einer gelblichen Flüssigkeit. Dieses Produkt hatte laut Gaschromatographie einen Gehalt von 71 %, entsprechend 15,3 g 100 %igem Produkt. Dies entsprach einer Ausbeute von 85 %, bezogen auf eingesetztes Isophoron. Das noch etwas feuchte Produkt konnte direkt in der zweiten Stufe eingesetzt werden.

2. Stufe

Wie in Beispiel 1.

Beispiel 4

1. Stufe

Eine Lösung von 28 g Isophoron in 250 g Ameisensäure wurde auf 0 °C abgekühlt. Durch diese Lösung wurde so lange ein Sauerstoffstrom mit ca. 4 % Ozon eingeleitet, bis die Lösung mit Ozon gesättigt war.

Dann wurde die ozonolysierte Lösung in 100 ml kochendes Wasser gegeben und 2 Stunden lang unter Rückfluss gekocht. Anschliessend wurde das Hydrolysat im Rotationsverdampfer konzentriert. Man erhielt 32,9 g einer gelben Flüssigkeit, die laut Gaschromatographie einen Gehalt von 81 % an 3,3-Dimethyl-5-oxo-hexansäure aufwies. Dies entsprach einer Ausbeute von 83,9 %, bezogen auf eingesetztes Isophoron.

Die Ameisensäure und das Wasser konnten aus dem rohen Produkt durch Destillation entfernt werden.

2. Stufe

Wie in Beispiel 1.

Beispiel 5

1. Stufe

Eine Lösung von 4 g Isophoron in 40 g Eisessig wurde bei 22 bis 30 °C ozonisiert. Nach der Ozonolyse wurden 20 ml Wasser zugegeben und die Lösung 3 Stunden lang auf 80 °C erwärmt und anschliessend am Vakuum eingedampft. Man erhielt 4,1 g einer gelben Flüssigkeit, die laut Gaschromatographie 81 % der 3,3-Dimethyl-5-oxo-hexansäure enthielt. Dies entsprach einer Ausbeute von 72 %, bezogen auf das eingesetzte Isophoron.

2. Stufe

Wie in Beispiel 1.

Beispiel 6

1. Stufe

Wie in Beispiel 1.

2. Stufe

In einem mit kaltem Wasser gekühlten Kolben wurde 22,5 g 85 %ige Phosphorsäure mit 14 g 43 %igem Wasserstoffperoxid vereinigt. Diese Mischung wurde auf 50 °C erwärmt. Dann wurden unter Rühren 4 g reine 3,3-Dimethyl-5-oxo-hexansäure in 3,5 g Wasser so eingetropft, dass die Temperatur zwischen 50 bis 55 °C blieb. Anschliessend wurde die Mischung über Nacht bei 55 °C weitergerührt.

Nach Einleiten von $SO_2$ zur Peroxidvernichtung wurde der pH-Wert mit konz. NaOH auf 1,5 eingestellt und dreimal mit 50 ml Aether extrahiert.

Nach Eindampfen der Lösung erhielt man einen Kristallbrei, der laut Gaschromatographie aus 2,5 g (als 100 %ig berechnet) 3,3-Dimethylglutarsäure bestand. Dies entsprach einer Ausbeute von 63,3 %, bezogen auf eingesetzte Dimethyl-oxo-hexansäure.

## Ansprüche

1. Verfahren zur Herstellung von 3,3-Dimethylglutarsäure aus Isophoron, dadurch gekennzeichnet, dass man in einer ersten Stufe Isophoron mit Hilfe von Ozon in ein Ozonanlagerungsprodukt überführt, dieses durch Hydrolyse in 3,3-Dimethyl-5-oxo-hexansäure überführt und letzteres in einer zweiten Stufe durch Behandeln mit Wasserstoffperoxid zur 3,3-Dimethylglutarsäure umsetzt.

2. Verfahren nach Anspruch 1, dadurch gekennzeichnet, dass man die erste Stufe kontinuierlich durchführt und die isolierte 3,3-Dimethyl-5-oxo-hexansäure in einer zweiten Stufe der Wasserstoffperoxidbehandlung unterwirft.

3. Verfahren nach Anspruch 1 bis 2, dadurch gekennzeichnet, dass man die Ozonolyse bei Temperaturen von − 80 bis + 40 °C durchführt und die Hydrolyse mit siedendem Wasser vornimmt.

4. Verfahren nach Anspruch 1 bis 3, dadurch gekennzeichnet, dass man die Ozonolyse und die Hydrolyse in Gegenwart von Lösungsmitteln durchführt.

5. Verfahren nach Anspruch 1 bis 4, dadurch gekennzeichnet, dass man die Umsetzung von 3,3-Dimethyl-5-oxo-hexansäure zur 3,3-Dimethylglutarsäure mittels Wasserstoffperoxid bei Temperaturen von 15 bis 70 °C durchführt.

6. Verfahren nach Anspruch 1 bis 5, dadurch gekennzeichnet, dass man die Wasserstoffperoxidbehandlung in Gegenwart starker Säuren durchführt.

## Claims

1. Process for the production of 3,3-dimethyl glutaric acid from isophorone, characterized in that in a first step isophorone is converted by means of ozone to a ozone addition product, said addition product is converted by hydrolysis to 3,3-dimethyl-5-oxo-hexanoic acid and the latter is reacted in a second step to 3,3-dimethyl glutaric acid by treatment with hydrogen peroxide.

2. Process according to claim 1, characterized in that the first step is carried out continuously and the isolated 3,3-dimethyl-5-oxo-hexanoic acid is subjected to the hydrogen peroxide treatment in a second step.

3. Process according to claims 1 and 2, characterized in that the ozonolysis is carried out at temperatures from − 80 to + 40 °C and the hydrolysis is effected by boiling water.

4. Process according to claims 1 to 3, characterized in that the ozonolysis and the hydrolysis are carried out in the presence of solvents.

5. Process according to claims 1 to 4, characterized in that the conversion of 3,3-dimethyl-5-oxo-hexanoic acid to 3,3-dimethyl glutaric acid by means of hydrogen peroxide is carried out at temperatures from 15 to 70 °C.

6. Process according to claims 1 to 5, characterized in that the hydrogen peroxide treatment is carried out in the presence of strong acids.

## Revendications

1. Procédé pour la préparation de l'acide 3,3-diméthylglutarique à partir de l'isophorone, caractérisé en ce que, dans une première étape, on transforme l'isophorone à l'aide d'ozone, en un produit d'addition de l'ozone, on transforme ce produit par hydrolyse en acide 3,3-diméthyl-5-oxo-hexanoïque et on fait réagir ce dernier dans une deuxième étape par traitement par le peroxyde d'hydrogène pour donner l'acide 3,3-diméthylglutarique.

2. Procédé selon la revendication 1, caractérisé en ce que l'on effectue la première étape en continu et on soumet l'acide 3,3-diméthyl-5-oxo-hexanoïque isolé au traitement par le peroxyde d'hydrogène dans une deuxième étape.

3. Procédé selon la revendication 1 ou 2, caractérisé en ce qu'on effectue l'ozonolyse à des températures de − 80 à + 40 °C et on effectue l'hydrolyse avec de l'eau bouillante.

4. Procédé selon la revendication 1 à 3, caractérisé en ce qu'on effectue l'ozonolyse et l'hydrolyse en présence de solvants.

5. Procédé selon la revendication 1 à 4, caractérisé en ce que la réaction de l'acide 3,3-diméthyl-5-oxo-hexanoïque pour donner l'acide 3,3-diméthylglutarique est effectuée au moyen de peroxyde d'hydrogène à des températures de 15 à 70 °C.

6. Procédé selon la revendication 1 à 5, caractérisé en ce que le traitement par le peroxyde d'hydrogène est effectué en présence d'acide fort.